# EUROPEAN PATENT SPECIFICATION

(11) **EP 0 907 627 B1**
(45) Date of publication and mention of the grant of the patent: **05.09.2001**
(21) Application number: 97927220.0
(22) Date of filing: 23.06.1997
(51) Int. Cl.: C07C 31/18, C07C 29/141, C13K 13/00

(54) **METHOD FOR PRODUCING XYLITOL**
VERFAHREN ZUR HERSTELLUNG VON XYLITOL
PROCEDE DE PREPARATION DE XYLITOL

(30) Priority: 24.06.1996 FI 962609
(43) Date of publication of application: 14.04.1999
(73) Proprietor: XYROFIN OY, 02150 Espoo (FI)
(72) Inventor: HEIKKILÄ, Heikki, FIN-02320 Espoo (FI); TYLLI, Matti, FIN-02460 Kantvik (FI); GOLDE, Martin, FIN-80101 Joensuu (FI); NYGREN, Johanna, FIN-08700 Virkkala (FI); EROMA, Olli-Pekka, FIN-48100 Kotka (FI)
(74) Representative: Syvänen, Ralf Ossian
(86) International application number: FI9700402
(87) International publication number: WO9749658

(56) References cited:
- EP-A- 0 694 515
- FR-A- 2 603 276
- US-A- 3 558 725

## Description

The invention relates to a method for producing xylitol.

Xylitol is a naturally occurring sugar alcohol that is obtained through the reduction of xylose and that has sweetness corresponding to "normal sugar" but a calorie content (2.4 kcal/kg) lower than in normal sugar. Xylitol is found in small amounts in a variety of fruits and vegetables and it is also formed in the human body as a normal metabolic product. Due to certain metabolic, dental and technical properties, xylitol is a very good special sweetener for different purposes, such as chewing gum, confectionery, etc. It can be mentioned as an example that xylitol metabolism is independent of insulin metabolism and therefore diabetics can also use xylitol. Xylitol also slows down bowel movement and it may therefore be of use in diets. It has also been found out that xylitol does not cause dental caries but it might even have an anticariogenic effect.

Despite the numerous advantages of xylitol, its use has been rather limited. This is due to the relatively high price of xylitol, which in turn results from difficulties in producing xylitol on a larger scale.

Xylitol has been previously prepared from xylan-containing material through hydrolysis. This results in a monosaccharide mixture containing xylose, for example. Xylose is then reduced to xylitol through catalytic reduction (hydrogenation) usually in the presence of a nickel catalyst, such as Raney nickel. The literature of the art describes several methods for producing xylose and/or xylitol from xylan-containing material. Examples include US Patent 3,784,408 (Jaffe et al), US Patent 4,066,711 (Melaja et al), US Patent 4,075,406 (Melaja et al), US Patent 4,008,285 (Melaja et al) and US Patent 3,586,537 (Steiner et al).

In several plants, majority of the hemicellulose is xylan that can be hydrolyzed into xylose. The primary starting material for xylan is the hemicellulose of deciduous trees that mainly consists of xylan. The use of xylan and xylose obtained as by-products of cellulose industry has recently also been the object of greater and greater attention. Xylose is formed, for example, in acid sulphite cookings where the typical bases include Mg²⁺, Ca²⁺, NH₄⁺ and Na⁺. The starting material may also be cooking liquor of neutral sulphite cookings after the xylo-oligomers of xylan have been hydrolyzed. In the cooking liquors of acid sulphite cookings, the hemicelluloses are already in a monosaccharide form. The term "cooking liquor" refers in this connection to a solution used in cooking or obtained after the cooking, or part thereof. The known catalytic methods for reducing xylose used in the production of xylitol usually require that the xylose to be reduced does not contain harmful impurities. The purification is very demanding and requires a multistep process (see US Patent 4,631,129, Heikkilä, and PCT/FI95/00224, Heikkilä et al) for example since the catalysts used in the xylose reduction reaction are very sensitive to impurities (see Härkönen, M. and Nuojua, P., Kemia-Kemi, no 3 (1980) pp 98-100). The purity of the final product is in turn greatly dependent on whether the xylitol can also be separated from the products produced during the reduction reaction.

When a sulphite cooking liquor is used as a raw material for xylose, the problem is the variation in the cooking conditions. Depending on the circumstances, hemicellulose of wood dissolves in different ways and produces greater or smaller amounts of xylose. In cooking conditions where only a small amount of xylose is produced, significant amounts of xylonic acid may also be formed. It is difficult to separate xylose found in such a product from a liquor containing xylonic acid for example by means of chromatography if the xylose is to be pure. The xylonic acid present in the solution makes the separation of xylose more difficult and therefore causes a decrease in the crystallization yield of xylose. However, it would be preferable to be able to use xylonic acid as a raw material for xylitol (see WO 93/1903, Vuorinen). Therefore, it would be desirable to provide a method by which xylose and xylonic acid obtained from sulphite cooking liquor, for example, could be converted together into xylitol.

As described above, the reduction of xylose to xylitol constitutes known technology. The reduction of xylonic acid, which is usually in the form of a lactone or a salt (see Paperi ja Puu, no 11, vol. 59 (1977) p 713), to xylitol is considerably more difficult, on the other hand. It has been found out that the simultaneous reduction of xylose and xylonic acid to xylitol is difficult since xylose decomposes under the strong reduction conditions required by xylonic acid.

The present invention relates to a method for producing xylitol. The method is characterized in that a mixture that contains xylose and xylonic acid or that is concentrated with respect to xylonic acid is reduced.

It has recently turned out that the reduction can be carried out catalytically or by using metal hydride reagents, such as sodium borohydride. The reduction is carried out suitably catalytically, in which case preferred catalysts include Raney-type catalysts and noble-metal catalysts, such as Ru, Pd and Pt, especially Ru.

A suitable catalytic reduction temperature is 70 to 150°C, preferably 100 to 130°C, and the reduction is carried out suitably at a pressure of 5000 to 20 000 kPa, preferably 10 000 to 13 000 kPa. The pH of the mixture to be reduced is preferably 0.5 to 3.5.

The mixture to be reduced is suitably a mixture obtained from sulphite cooking liquor by extraction, or a fraction obtained by chromatographic separation, preferably through the use of a simulated moving bed (SMB) (see WO 94/26380), or runoff formed in the crystallization of a xylose fraction. The fractions that have been separated before the reduction must be possibly purified, for example, by ion exchange. The xylose and xylonic acid fractions may require further purification by neutralization/precipitation/filtration and/or treatment with carbon or an adsorbent. It is also possible to use two-step hydrogenation, which means that in the first step the hydrogenation is carried out for instance with a Raney nickel catalyst and in the second step with a noble-metal catalyst, for example. A manner of implementing the invention is to carry out preoxidation, in which case the separation of the xylonic acid concentrate from the solution becomes more effective. Xylitol prepared according to the invention can be preferably isolated from the product solution of the reduction chromatographically or preferably by crystallization.

The method according to the invention makes it possible to considerably reduce the costs of producing xylitol. The method enables for example better use of the raw material (the raw material is sufficient for as much as about twofold amount of xylitol).

The following examples illustrate the invention.

### Example 1

Hydrogenation of an acid fraction obtained from the chromatographic separation of sulphite cooking liquor

By using a simulated moving bed (SMB) (WO 94/26380) a fraction was separated chromatographically from an acid Mg-sulphite cooking liquor of beech tree and it was purified with granulated (Chemviron CPG™) carbon, a strongly acid cation-exchange resin (DOW 88™, in an acid form) and with a slightly basic anion-exchange resin (DOW 66™, in a basic form). The conditions of the run were: a flow of 1.0 bv/h/column (bv = bulk volume), a temperature of 40°C and a dry solids content of the feed solution of 23%. The reaction was carried out in a 5-litre Medimex autoclave (batch-type reactor) at 100°C, at a pressure of 100 bar and at a pH of 1.3 by using as a catalyst ruthenium/carbon (5% Ru on carbon, Engelhard CP 56 × L/R/WW), the dosage (Ru/C) of which was 13% of the dry matter of the solution. The time of hydrogenation was four hours. The feed and product analyses of the hydrogenation are shown in Table 1.

### Example 2

Hydrogenation of a product fraction obtained from chromatographic separation

A fraction obtained in the manner described in Example 1 was purified in the following manner. The fraction was neutralized to a pH value of 5 by adding 7.5% calcium hydroxide calculated on the dry matter of the fraction. The precipitate was filtered off. The filtrate was purified with a strong cation-exchange resin and with a slightly basic anion-exchange resin as in Example 1. The hydrogenation was carried out under the same conditions as in Example 1. The feed and product analyses are shown in Table 1.

### Example 3

Reduction of runoff from xylose crystallization

The runoff was purified with a strongly acid cation-exchange resin and a slightly basic anion-exchange resin as described in Example 1. The hydrogenation was carried out under the same conditions as in Examples 1 and 2. The feed and product analyses are shown in Table 1.

### Example 4

Hydrogenation of a mixture (runoff/acid = 80/20%) of the acid fraction from the chromatographic separation of sulphite cooking liquor and the runoff from xylose crystallization.

The mixture was purified with a strongly acid cation-exchange resin and a slightly basic anion-exchange resin as described in Example 1. The hydrogenation was carried out under the same conditions as in Examples 1 to 3. The feed and product analyses are shown in Table 1.

### Example 5

Reduction of a product fraction from the chromatographic separation of sulphite cooking liquor by using two-step hydrogenation

The prehydrogenation was carried out on a non-purified SMB separation product under the following conditions. The catalyst was Raney nickel (10% of the dry solids content of the solution, Chemcat J 10 GS), the temperature was 100°C, the pressure was 4000 kPa (at the end the pressure was raised to 8000 kPa), and the time of hydrogenation was four hours. Cations were removed from the product of hydrogenation with a strongly acid cation-exchange resin (DOW 88, in an acid form), in other words the acid was liberated from its salt. The flow rate was 2 bv/h, the temperature was 40°C and the dry solids content of the feed was about 22%. The actual hydrogenation was carried out under the same conditions as the hydrogenations in Examples 1 to 4. The feed (feed of the first stage) and product analyses (= product of the second stage) are shown in Table 1.

**Table 1**

| Hydrogenation of xylose-xylonic acid mixtures | | | |
|---|---|---|---|
| Feed solution of hydrogenation | Components | Feed (% by wt of dry matter) | Product (% by wt of dry matter) |
| Example 1 | xylose | 14 | 0 |
| Acid fraction from | xylitol | 0 | 40 |
| separation | xylonic acid | 40 | no analysis |
| Example 2 | xylose | 53 | 0 |
| Product fraction | xylitol | 0 | 74 |
| from separation | xylonic acid | 24 | 1 |
| Example 3 | xylose | 30 | 0 |
| Runoff from xylose | xylitol | 0 | 58 |
| crystallization | xylonic acid | 35 | 11 |
| Example 4 | xylose | 29 | 0 |
| Mixture of runoff | xylitol | 0 | 40 |
| and acid fraction 80:20 | xylonic acid | 31 | 26 |
| Example 5 | xylose | 50 | 0 |
| Product fraction | xylitol | 0 | 71 |
| from separation Double hydrogenation | xylonic acid | 25 | no analysis |

### Example 6

### Preoxidation

2 g of MgO, 62 g of sulphite cooking liquor corresponding to Example 1, and 140 g of SO₂ solution (concentration 70 to 72 g SO₂/l) were added into autoclaves. The autoclaves were closed and put into a glycerol bath at 150°C. The autoclaves were kept in the bath for 30 min, 1 h, 2 h and 4 h, whereafter they were cooled. The solution was filtered and analyzed. 28% of the xylose was oxidized into xylonic acid in four hours (Table 2).

**Table 2**

| Oxidation of xylose into xylonic acid | | |
|---|---|---|
| Time | Xylose g/l | Xylonic acid g/l |
| 0 | 20.1 | 10.0 |
| 30 min | 15.8 | 10.7 |
| 1 h | 11.7 | 13.9 |
| 2 h | 7.7 | 15.7 |
| 4 h | 5.7 | 15.4 |

### Example 7

### Hydrogenation of a mixture concentrated with respect to xylonic acid

The hydrogenation was carried out in a five-litre Medimex autoclave (batch-type reactor) at 110°C and at a pressure of 13 000 kPa by using as a catalyst Ru/carbon (5% Ru on carbon, Engelhard CP 56 × L/R/WW) the dosage of which was 18% of the dry matter. The time of hydrogenation was 3 hours. Table 3 shows the compositions of the starting material and the obtained product.

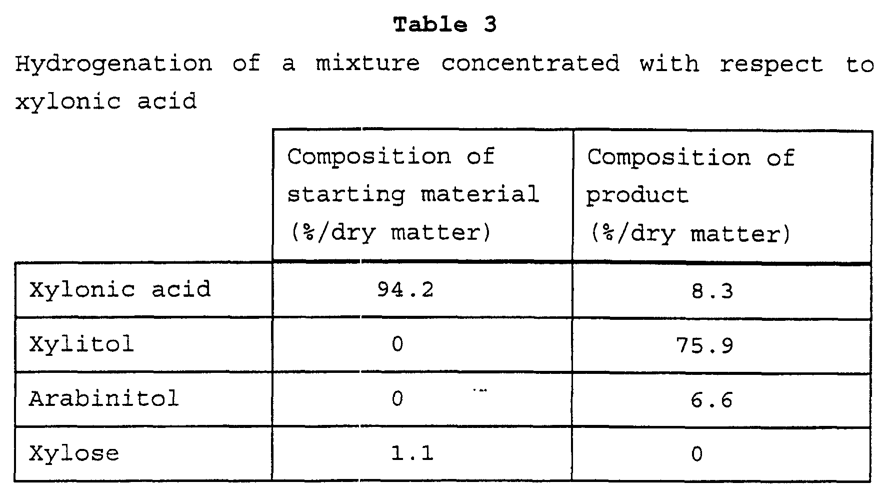

### Example 8

### Hydrogenation with Raney nickel of a mixture concentrated with respect to xylonic acid

166 g/l of xylonic acid in 70% methanol was hydrogenated in an autoclave with Raney nickel (2 g) at 122°C and at a pressure of 6500 kPa for 18 hours. The results are shown in Table 4.

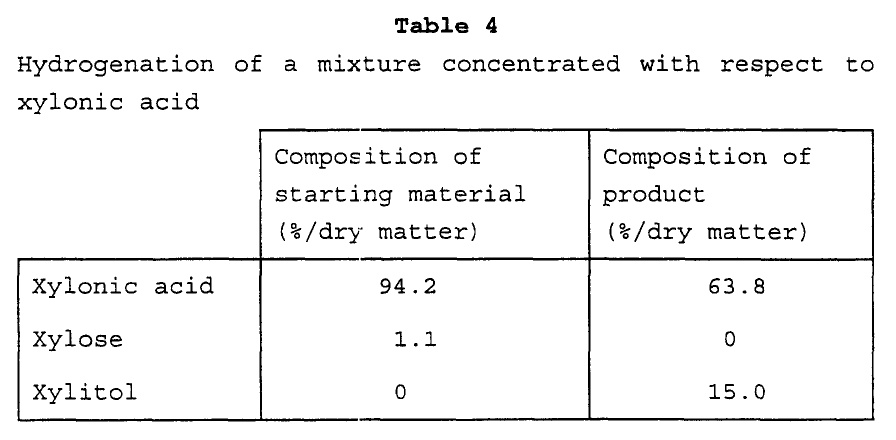

### Example 9

### Hydrogenation with rhodium of a mixture concentrated with respect to xylonic acid

166 g/l of xylonic acid in water was hydrogenated in an autoclave by using as a catalyst 0.17 g of 5% Rh/2% Mo/Al₂O₃ at 140°C and at a pressure of 6500 kPa for 18 hours. The results are shown in Table 5.

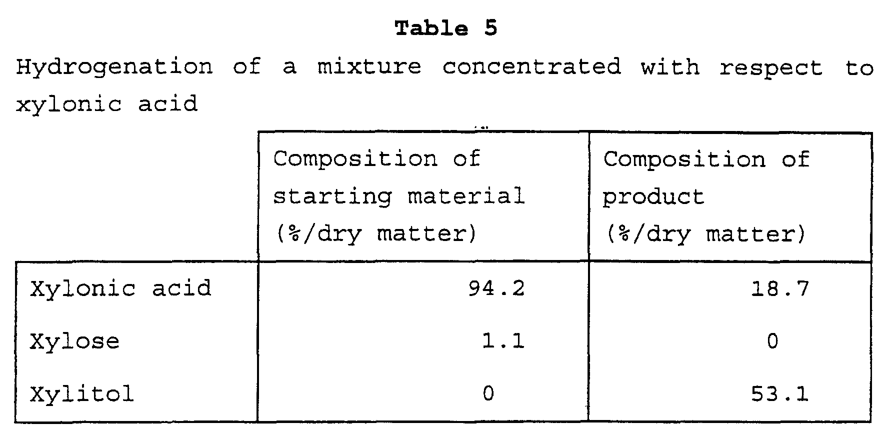

### Example 10

### Reduction with sodium borohydride of an oxidized xylose-xylonic acid fraction produced by chromatographic separation

The reaction was carried out at a normal pressure and at room temperature by mixing. The reaction time was 2 hours after the addition of the reagent (added gradually). The fraction was reduced as such and after cation exchange at a dry solids content of about 10%. Sodium borohydride was added in the ratio of 3 g/100 g of the natural weight of the solution (= 10 g of dry matter). The sodium borohydride was added in the form of 4% aqueous solution. The reaction was terminated with 6 N hydrochloric acid by acidifying the solution (pH 2). The results are shown in Table 6.

**Table 6**

| Reduction of an oxidized xylose-xylonic acid fraction with sodium borohydride | | | |
|---|---|---|---|
| | xylose (%/dry matter) | acid (%/dry matter) | xylitol (%/dry matter) |
| Acid fraction | 1.7 | 7.5 | 0 |
| Reduced acid fraction | 0.2 | 3.1 | 2.1 |
| Acid fraction + cation exchange | 1.9 | 8.1 | 0 |
| Reduced fraction + cation exchange | 0.6 | 3.2 | 2.4 |

### Example 11

### Reduction of a synthetic xylose-xylonic acid mixture with sodium borohydride

The reduction was carried out under the same conditions as in Example 8, except that the feed solution was a synthetic xylose-xylonic acid mixture. The results are shown in Table 7.

**Table 7**

| Reduction of a synthetic xylose-xylonic acid with sodium borohydride | | | |
|---|---|---|---|
| | xylose (% dry matter) | acid (% dry matter) | xylitol (%/dry matter) |
| Mixture before reduction | 50.7 | 46.5 | 0 |
| Mixture after reduction | 0.1 | 31.6 | 70.1 |

### Example 12

### Crystallization of xylitol

The raw material was xylitol prepared as described in Example 5. 97 g of xylitol (refractometric dry solids content RDS 11.4%, feed solution) was evaporated into an RDS content of 91.4% at 60°C. The mass was transferred to a 1-litre reaction vessel where it was seeded with 0.06 g of xylitol crystals at 60°C. A linear 49-hour cooling program (60.5°C to 30°C) was activated. After the cooling, the temperature of the mass was increased by about 3°C and the mass was centrifuged. The xylitol purity of the mass was 77% on the dry matter. The crystals were separated with a centrifuge (basket diameter 22 cm, mesh size 0.15 mm) at 4500 rpm for 5 minutes, and the crystals were washed. The yield was 30 g of dried crystals. The xylitol purity of the crystals was 81.2% on dry matter basis.

### Example 13

### Crystallization of xylitol

The raw material was xylitol prepared as described in Example 2. The xylitol solution was filtered through a membrane of 12 *µ*m. 170 g (dry matter) of xylitol (RDS 19.7%, feed solution) was evaporated into an RDS content of 91.3% at 60°C. The mass was transferred to a 1-litre reaction vessel where it was seeded with 0.05 g of xylitol crystals at 60°C. A linear 41-hour cooling program (60.5°C to 30°C) was activated. After the cooling, the temperature of the mass was increased by about 3°C and the mass was centrifuged. The xylitol purity of the mass was 64.3% of the dry matter. The crystals were separated with a centrifuge (basket diameter 22 cm, mesh size 0.15 mm) at 4500 rpm for 5 minutes and washed. The yield was 54 g of dried crystals. The xylitol purity of the crystals was 93.3% on dry matter basis.

### Example 14

### Crystallization of xylitol

The raw material was xylitol prepared as described in Example 3. The xylitol solution was filtered through a membrane of 12 *µ*m. 185 g (dry matter) of xylitol (RDS 20.9%, feed solution) was evaporated into an RDS content of 92.2% at 60°C. The mass was transferred to a 1-litre reaction vessel where it was seeded with 0.05 g of xylitol crystals at 56.5°C. A linear 69-hour cooling program (57°C to 30°C) was activated. After the cooling, the temperature of the mass was increased by about 3°C and the mass was centrifuged. The xylitol purity of the mass was 56.5% of the dry matter. The crystals were separated with a centrifuge (basket diameter 22 cm, mesh size 0.15 mm) at 4500 rpm for 5 minutes and washed. The yield was 55 g of dried crystals. The xylitol purity of the crystals was 68.0% on dry matter basis.

### Example 15

### Separation of xylose and xylonic acid from Ca-sulphite cooking liquor by ethanol extraction

Commercially available dry and powdery Ca-sulphite cooking liquor of a deciduous tree, the composition of which is shown in Table 8 (1), was extracted with ethanol. The amount of powder in the extraction was 1500 g and the amount of 95% ethanol was 15 1. The mixture was mixed at 50°C for 4 hours, whereafter it was filtered and the obtained cake was dried. The amount of the dissolved solids was 32%. The filtrate was evaporated in a rotavapor at a decreased pressure. The evaporation residue was dissolved in about 8 litres of water. The composition of the solution is shown in Table 8 (2). The xylose yield was about 78% and the xylonic acid yield was about 43%. The yields increased to 95% and 56%, respectively, when the ethanol extraction was repeated.

**Table 8**

| Separation of xylose and xylonic acid by ethanol extraction. | | | |
|---|---|---|---|
| | | Starting material 1 | Extract 2 |
| Dry solids content | (g/100 g)* | 96 | 21 |
| Neutral oligosaccharides | (% of dry matter) | 1.1 | 2.6 |
| Glucose | (% of dry matter) | 1.5 | 2.8 |
| Xylose | (% of dry matter) | 12.5 | 24.1 |
| Galactose-rhamnose | (% of dry matter) | 1.6 | 3.0 |
| Mannose | (% of dry matter) | 1.1 | 1.9 |
| Xylonic acid | (% of dry matter) | 5.2 | 5.4 |

| | | | |
|---|---|---|---|
| * The dry solids content of the powder was determined with the K. Fischer method and the dry solids content of the solution was determined refractometrically by utilizing the refractive index table for pure xylose. | | | |

### Example 16

### Prepurification of a xylose-xylonic acid fraction

The feed solution was a xylose-xylonic acid fraction from a continuous chromatographic separation process. The fraction was supplied to a series of ion exchangers comprising a strongly acid cation-exchange resin (DOW 88™) and two slightly basic anion-exchange resins (DOW 66™). Cations adhere to the cation-exchange resin and converted into a free acid, whereafter the anions adhere to the anion-exchange resin.

The dry solids content of the feed solution was 32%, the temperature was 40°C and the flow rate was 2 bv/h/column. In this experiment, the solution was treated in an amount approximately corresponding to the total resin volume.

In such a run, xylonic acid adhered to both anion-exchange resins: 22 g/l of resin to the first one and 63 g/l of resin to the second one. The feed and product analyses are shown in Table 9.

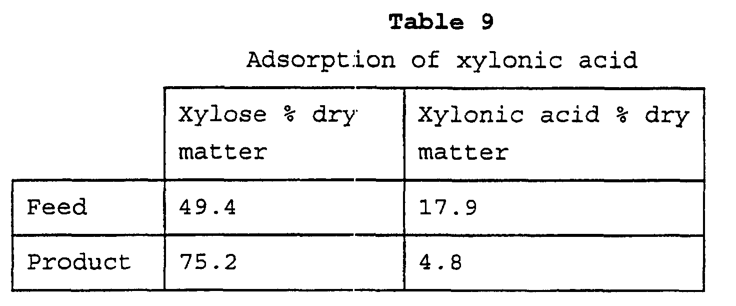

### Example 17

### Prepurification of a xylose-xylonic acid fraction

Chromatographic separation of Mg-sulphite cooking liquor was carried out by using a slightly acid cation-exchange resin, Finex CA 24 GC™. The temperature was 65°C and the flow rate was 0.19 m/h. The pH of the feed solution was 1.2 and the xylose content was 9.8%. The xylose fraction capacity with the fraction purity of 25% was 9.6 kg of dry matter/m³/h and the maximum purity of xylose in the separation was 31.4%. The xylonic acid content in the feed solution was 5.5%/dry matter (RDS) and in the xylose fraction 16.7%/dry matter. The order of elution was: majority of the salt followed by xylose and xylonic acid almost simultaneously (xylonic acid slightly later).

### Example 18

### Prepurification of a xylose-xylonic acid fraction

Chromatographic separation of Mg-sulphite cooking liquor was carried out by using a slightly acid cation-exchange resin, Purolite C 105™. The temperature was 65°C and the flow rate was 0.7 m/h. The pH of the feed solution was 4.5 and the xylose content was 10.9%. The xylose fraction capacity with the fraction purity of 25% was 19.0 kg of dry matter/m³/h and with the fraction purity of 40% it was 7.8 kg of dry matter/m³/h and the maximum purity of xylose in the separation was 42.7%. The xylonic acid content in the feed solution was 5.6%/dry matter (RDS). The xylonic acid purity in the xylose fraction having fraction purity of 25%/dry matter of xylose was 11.7%/dry matter, and in the xylose fraction having fraction purity of 40%/dry matter of xylose the xylonic acid purity was 18.5%/dry matter. The salts, xylose and xylonic acid were eluted almost simultaneously (xylonic acid slightly later).

### Example 19

### Prepurification of a xylose-xylonic acid fraction

Chromatographic separation of Mg-sulphite cooking liquor was carried out by using a fibre-like (staple fibre) cation-exchange resin, Smoptec 101,3™, polystyrene skeleton, that is activated with sulphonic acid. The temperature was 65°C and the flow rate was 1.8 m/h. The pH of the feed solution was 2.2 and the xylose content was 8.9%. The maximum purity of xylose in the separation was 23.4%. The xylonic acid content in the feed solution was 5.1%/dry matter (RDS) and the maximum purity in the xylose fraction was 15.0%/dry matter. The order of elution was: majority of the salt, and xylose and xylonic acid together.

### Example 20

### Prepurification of a xylose-xylonic acid fraction

Chromatographic separation of Mg-sulphite cooking liquor was carried out by using a strongly acid cation-exchange resin, Finex CS 11 GC™. The temperature was 65°C and the flow rate was 0.7 m/h. The pH of the feed solution was 1.0 and the xylose content was 11.9%. The xylose fraction capacity with the fraction purity of 40% was 11.2 kg of dry matter/m³/h and the maximum purity of xylose in the separation was 44.8%. The xylonic acid content in the feed solution was 5.5%/dry matter (RDS) and in the xylose fraction 25%/dry matter. The order of elution was: salt, xylose and xylonic acid. The two latter fractions partly overlapped.

## Claims

1. A method for producing xylitol, **characterized in that** a mixture that contains xylose and xylonic acid or that is concentrated with respect to xylonic acid is reduced, and xylitol is recovered.

2. A method according to claim 1, **characterized in that** the reduction is carried out catalytically.

3. A method according to claim 2, **characterized** by using a noble-metal catalyst.

4. A method according to claim 2, **characterized in that** the catalyst is Ru, Pd, Raney-Ni or Rh.

5. A method according to claim 4, **characterized in that** the catalyst is Ru.

6. A method according to any one of the preceding claims, **characterized in that** the reduction is carried out at 80°C to 130°C.

7. A method according to any one of the preceding claims, **characterized in that** the reduction is carried out at a pressure of 10 000 to 13 000 kPa.

8. A method according to any one of the preceding claims, **characterized in that** the reduction is carried out at a pH of 0.5 to 3.5.

9. A method according to claim 1, **characterized in that** the reduction is carried out by utilizing a metal hydride reagent.

10. A method according to claim 9, **characterized in that** the metal hydride reagent is sodium borohydride.

11. A method according to any one of the preceding claims, **characterized in that** the mixture to be reduced is a fraction that is obtained from cooking liquor of a sulphite cook by chromatographic separation.

12. A method according to claim 11, **characterized in that** the chromatographic separation is carried out by using a simulated moving bed (SMB).

13. A method according to any one of claims 1 to 10, **characterized in that** the mixture to be reduced is a mixture that is obtained from cooking liquor of a sulphite cook by extraction.

14. A method according to any one of claims 11 to 13, **characterized in that** the sulphite cook is an Mg or Ca-sulphite cook.

15. A method according to any one of claims 1 to 10, **characterized in that** the mixture to be reduced is runoff formed in the crystallization of xylose.

16. A method according to any one of the preceding claims, **characterized in that** the mixture to be reduced is concentrated with respect to xylonic acid by ion exchange.

17. A method according to any one of the preceding claims, **characterized in that** the xylose in the mixture to be reduced is at least partly oxidized into xylonic acid.

18. A method according to claim 17, **characterized in that** the mixture to be reduced is oxidized with bisulphite.

19. A method according to any one of the preceding claims, **characterized in that** xylitol is separated by crystallization.

20. A method according to any one of claims 1 to 18, **characterized in that** xylitol is separated by chromatographic separation.

## Patentansprüche

1. Verfahren zur Herstellung von Xylitol, dadurch gekennzeichnet, daß eine Mischung, die Xylose und Xylonsäure enthält oder die in bezug auf Xylonsäure konzentriert ist, reduziert wird und Xylitol gewonnen wird.

2. Verfahren nach Anspruch 1, dadurch gekennzeichnet, daß die Reduktion katalytisch ausgeführt wird.

3. Verfahren nach Anspruch 2, gekennzeichnet durch die Verwendung eines Edelmetallkatalysators.

4. Verfahren nach Anspruch 2, dadurch gekennzeichnet, daß der Katalysator Ru, Pd, Raney-Ni oder Rh ist.

5. Verfahren nach Anspruch 4, dadurch gekennzeichnet, daß der Katalysator Ru ist.

6. Verfahren nach einem der vorangehenden Ansprüche, dadurch gekennzeichnet, daß die Reduktion bei 80°C bis 130°C durchgeführt wird.

7. Verfahren nach einem der vorangehenden Ansprüche, dadurch gekennzeichnet, daß die Reduktion bei einem Druck von 10000 bis 13000 kPa durchgeführt wird.

8. Verfahren nach einem der vorangehenden Ansprüche, dadurch gekennzeichnet, daß die Reduktion bei einem pH-Wert von 0,5 bis 3,5 durchgeführt wird.

9. Verfahren nach Anspruch 1, dadurch gekennzeichnet, daß die Reduktion unter Verwendung eines Metallhydridreagens durchgeführt wird.

10. Verfahren nach Anspruch 9, dadurch gekennzeichnet, daß das Metallhydridreagens Natriumborhydrid ist.

11. Verfahren nach einem der vorangehenden Ansprüche, dadurch gekennzeichnet, daß die zu reduzierende Mischung eine Fraktion ist, die von einer Kochlösung einer Sulphitkochung durch chromatographische Trennung erhalten wird.

12. Verfahren nach Anspruch 11, dadurch gekennzeichnet, daß die chromatographische Trennung unter Verwendung eines simulierten Fließbettes (SFB) durchgeführt wird.

13. Verfahren nach einem der Ansprüche 1 bis 10, dadurch gekennzeichnet, daß die zu reduzierende Mischung eine Mischung ist, die von der Kochlösung einer Sulphitkochung durch Extraktion gewonnen wird.

14. Verfahren nach einem der Ansprüche 11 bis 13, dadurch gekennzeichnet, daß die Sulphitkochung eine Mg- oder Ca-Sulphitkochung ist,

15. Verfahren nach einem der Ansprüche 1 bis 10, dadurch gekennzeichnet, daß die zu reduzierende Mischung ein Ablauf ist, der bei der Kristallisation von Xylose entsteht.

16. Verfahren nach einem der vorangehenden Ansprüche, dadurch gekennzeichnet, daß die zu reduzierende Mischung in bezug auf Xylonsäure durch Ionenaustausch konzentriert ist.

17. Verfahren nach einem der vorangehenden Ansprüche, dadurch gekennzeichnet, daß die Xylose in der zu reduzierenden Mischung zumindest teilweise zu Xylonsäure oxidiert ist.

18. Verfahren nach Anspruch 17, dadurch gekennzeichnet, daß die zu reduzierende Mischung mit Bisulphit oxidiert ist.

19. Verfahren nach einem der vorangehenden Ansprüche, dadurch gekennzeichnet, daß Xylitol durch Kristallisation abgetrennt wird.

20. Verfahren nach einem der Ansprüche 1 bis 18, dadurch gekennzeichnet, daß Xylitol durch chromatographische Trennung abgetrennt wird.

## Revendications

1. Procédé de préparation de xylitol, caractérisé en ce que l'on réduit un mélange qui contient du xylose et de l'acide xylonique ou qui est concentré en acide xylonique, et on récupère du xylitol.

2. Procédé selon la revendication 1, caractérisé en ce que l'on effectue la réduction avec catalyse.

3. Procédé selon la revendication 2, caractérisé en ce que l'on utilise un catalyseur au métal noble.

4. Procédé selon la revendication 2, caractérisé en ce que le catalyseur est du Ru, du Pd, du Ni Raney ou du Rh.

5. Procédé selon la revendication 4, caractérisé en ce que le catalyseur est du Ru.

6. Procédé selon l'une quelconque des précédentes revendications, caractérisé en ce que l'on effectue la réduction à une température de 80 à 130 °C.

7. Procédé selon l'une quelconque des précédentes revendications, caractérisé en ce que l'on effectue la réduction sous une pression de 10 000 à 13 000 kPa.

8. Procédé selon l'une quelconque des précédentes revendications, caractérisé en ce que l'on effectue la réduction à un pH de 0,5 à 3,5.

9. Procédé selon la revendication 1, caractérisé en ce que l'on effectue la réduction en utilisant un réactif hydrure métallique.

10. Procédé selon la revendication 9, caractérisé en ce que le réactif hydrure métallique est le borohydrure de sodium.

11. Procédé selon l'une quelconque des précédentes revendications, caractérisé en ce que le mélange à réduire est une fraction qui est obtenue à partir d'une lessive de cuisson d'une cuisson au sulfite, par séparation chromatographique.

12. Procédé selon la revendication 11, caractérisé en ce que l'on effectue la séparation chromatographique en utilisant un lit mobile simulé (SMB).

13. Procédé selon l'une quelconque des revendications 1 à 10, caractérisé en ce que le mélange à réduire est un mélange qui est obtenu à partir d'une lessive de cuisson d'une cuisson au sulfite, par extraction.

14. Procédé selon l'une quelconque des revendications 11 à 13, caractérisé en ce que la cuisson au sulfite est une cuisson au sulfite de Mg ou de Ca.

15. Procédé selon l'une quelconque des revendications 1 à 10, caractérisé en ce que le mélange à réduire est le sous-produit rejeté formé dans la cristallisation de xylose.

16. Procédé selon l'une quelconque des revendications précédentes, caractérisé en ce que le mélange à réduire est concentré en acide xylonique par échange d'ions.

17. Procédé selon l'une quelconque des revendications précédentes, caractérisé en ce que le xylose dans le mélange à réduire est au moins partiellement oxydé en acide xylonique.

18. Procédé selon la revendication 17, caractérisé en ce que le mélange à réduire est oxydé avec du bisulfite.

19. Procédé selon l'une quelconque des revendications précédentes, caractérisé en ce que l'on sépare le xylitol par cristallisation.

20. Procédé selon l'une quelconque des revendications 1 à 18, caractérisé en ce que l'on sépare le xylitol par séparation chromatographique.
